# EUROPEAN PATENT APPLICATION

(11) **EP 2 382 964 A1**
(43) Date of publication of application: **02.11.2011**
(21) Application number: 10425369.5
(22) Date of filing: 03.12.2010
(51) Int. Cl.: A61K 8/73, A61K 8/99, A61Q 11/00

(54) **Method of obtaining a preparation of biological origin for oral hygiene to prevent the onset of tooth decay**

(30) Priority: 04.12.2009 IT RM20090643
(71) Applicant: ABR Consulting S.r.l., 63100 Ascoli Piceno (AP) (IT)
(72) Inventor: Carucci, Andrea, 00178 Roma (IT); Casini, Simonetta, 00176 Roma (IT)
(74) Representative: Cardelli, Guido

(57) **Abstract**

A method of obtaining a preparation of biological origin for oral hygiene to prevent the onset of tooth decay, comprises steps of fermentation of bacteria in a culture medium, separation of the same and purification of the culture medium in which the bacteria have been cultivated, the step of fermentation of bacteria including a combined addition of starch and cellulose to the culture medium to the final concentration in the culture medium between 0.2 g/l and 2 g/l, and preferably of 0.5 g/l each.

## Description

The invention relates to a method of obtaining a preparation of biological origin for oral hygiene to prevent the onset of tooth decay.

The progression of tooth decay occurs through three different steps:
- the *Streptococcus mutans* cariogenic bacterium growing in the oral cavity produces an extra-cellular polymer being constituted of a polysaccharide chain able to adhere to the tooth surface;
- then bacterial plaque, i.e. the bacterial colonisation of the polysaccharide produced, is formed; finally,
- caries occurs, i.e. the demineralisation of the enamel due to bacterial metabolites able to erode the tooth surface, causing a carious lesion.

The problem of removing the bacterial plaque in order to prevent the onset of tooth decay has been faced with various means, such as mechanical removal e.g. by brushing the teeth, inhibition of the bacterial growth by using bactericidal substances, chemical removal by means of surfactant agents or other chemical substances by direct action.

The present invention aims to face said problem through the break-down of the polysaccharide produced by the *Streptococcus mutans* cariogenic bacterium using a preparation of biological origin from bacterial culture.

The bacteria of genus *Bacillus* that are grown in suitable culture media (C.R. Harwood and S.M. Cutting, "Molecular Biological Methods for Bacillus", John Wiley & Sons 1990, 1-21), express a series of extra-cellular enzymes able to act on different types of polysaccharides like e.g. starches, cellulose, laminarin, pullulan, xylan.

The bacteria of genus *Bacillus* are also able to act on the biofilm produced by *Streptococcus mutans* bacterium. An article describes how a strain similar to *Lactobacillus fermentum* can prevent the formation of insoluble glucan (J. Chung, E.-S. Ha, H.-R. Park, S. Kim. (2004) Isolation and characterization of Lactobacillus species inhibiting the formation of Streptococcus mutans biofilm. Oral Microbiology and Immunology 19:3, 214-216).

The Patent application US 2002/006385 A1 discloses an oral composition constituted of starch-hydrolysing and starch-modifying enzymes like e.g. alpha-amylase and cyclodextrine glycosyltransferase (CGTase).

The Patent application WO 98/57653 discloses an oral composition comprising a pullulanase and a dextranase.

The Patent application JP 55 088693 A1 discloses enzymes produced by microorganisms like *Bacillus circulans* that hydrolyse insoluble alpha-glucan.

The Patent application WO 98/16190 A discloses alpha-amylase to which has been added by cloning the domain SBD, isolated by *Aspergillus niger,* for increasing the enzymatic activity.

The Patent US 5,306,639 discloses the expression and the secretion of a glucan hydrolase, i.e. an enzyme able to hydrolyse the glucan by recombinant DNA technology.

In the scientific literature it is known that it is possible to induce the bacteria contained in bacterial cultures, by adding to the latter, one or more substances to produce a compound able to act on determined substrates. An example of this kind of process is exposed in the Patent EP 0 704 202 A1 in which there is described the production of cycloisomaltooligosaccharides by a *Bacillus* grown in dextran, able to inhibit the glucan synthetase of *Streptococcus mutans* Generally all the bacteria including *Bacilli* must be cultivated in culture media constituted of basic elements and compounds added for the purpose of enriching the medium (peptones, yeast extracts, hydrolysates from meat, etc.). A micro-organism for its growth needs sources of carbon, hydrogen, oxygen and nitrogen as essential elements, and others elements are added only in traces, like Fe, Ca, Co, Mn, and act as enzymatic co-factors in some reactions. An object of the present invention is to obtain a preparation of biological origin able to break-down the polysaccharide chain produced by the *Streptococcus mutans* bacterium, so avoiding the formation of the plaque and preventing the bacterial metabolites to erode the teeth surface with consequent carious lesion. This object is achieved by providing a method of obtaining a preparation of biological origin for oral hygiene to prevent the onset of tooth decay, comprising steps of fermentation of bacteria in a culture medium, separation of the same and purification of the culture medium in which the bacteria have been cultivated, the step of fermentation of bacteria including a combined addition of starch and cellulose to the culture medium to the final concentration in the culture medium between 0.2 g/l and 2 g/l.

The method according to the present invention originates from the observation of the inventors that through the fermentation of a bacterium of genus *Bacillus* (*clausii, cereus, subtilis*) in culture media added with a starch such as rice starch, corn starch, soluble starch, potato starch, and of cellulose, for example microcrystalline cellulose, methyl cellulose, carboxymethyl cellulose, ethyl cellulose, a growth broth is obtained that contains a preparation having a breaking-down activity on the polysaccharide chain produced by the *Streptococcus mutans* bacterium.

The breaking-down activity of the samples is demonstrated both on a suspension of *S. mutans* and on extracted, cleaned and sterilised teeth, and is proved by a colorimetric test for determining the reducing sugars and by High Performance Liquid Chromatography (HPLC).

However, as observed by the inventors, for obtaining such a preparation by fermentation of the same bacteria (*Bacillus clausii, cereus, subtilis,* ecc.) in commercial culture media, even if added with starch and cellulose, complex purification processes are requested, as several steps are necessary with consequent great cost increase and high decreases in the process yield that advise against the use of the commercial culture media.

The inventors found that, by replacing the commercial culture media with *Saccharomyces cerevisiae* (brewer's yeast), a preparation is obtained that needs only a step of ultrafiltration and a single chromatographic pass, and this makes the process cheap and adapted to be industrialised (Table 1).

**Table 1**

| MEDIA | No. of PURIFICATION PASSES | TOTAL UNITS per litre of fermentation | PROCESS YIELD |
|---|---|---|---|
| *S. cerevisiae* | 2 | 800 | 60% |
| Simple broth (Peptone water) | 6 | 77 | 5.7% |
| BaCtO™ Soytone | 6 | 81 | 6% |
| Commercial yeast Extract | 6 | 69 | 5.2% |
| Tryptic Soy Broth | 6 | 54 | 4% |

| | | | |
|---|---|---|---|
| Bacto™ Soytone is a commercial preparation of soy enzymatic hydrolysate. | | | |

In the inventors' opinion the preparation obtained by the process according to the present invention is a polypeptide mixture, that is not comprised of proteins, that has no activity on starch and cellulose, that has breaking-down activity on and not directly inhibits the polysaccharide chain produced by the *Streptococcus mutans,* with the meaning that the preparation breaks down the polysaccharide chain, by decomposing it in reducing sugars. Further, the inventors observed that the addition in the culture medium of only one of the two components between starch, such as soluble starch, rice starch, corn starch, potato starch, and cellulose, for example microcrystalline cellulose, methyl cellulose, carboxymethyl cellulose, ethyl cellulose, does not allow the desired preparation to be obtained.

After studying mixtures in different amounts between 0.2 g/l and 2 g/l, optimal amount of starch and cellulose to be used in the culture medium in the method according to the invention has been found equal to 0.5 g/l for each of the two components.

Some embodiments of the method of the invention are given below in the form of examples, with reference also to the enclosed figures, in which:
figure 1 is a HPLC chromatogram to checking any glucose in a sample that is negative to the colorimetric test;
figure 2 a HPLC chromatogram to checking any glucose in a sample that is positive to the colorimetric test; and
figures 3 and 4 are photographs that show the condition of a tooth before being treated with a preparation according to the invention and after a treatment of five min, respectively.

### EXAMPLE 1

In this first example a process of production and purification of a preparation from *Bacillus clausii* obtained according to the invention is described.

The whole method is divided in three steps: fermentation, extraction and purification.

The materials of which the fermentation medium is formed are:
*Saccharomyces cerevisiae* (block of brewer's yeast) 25 g;
NaCl 5 g ;
Soluble starch 0.5 g;
Microcrystalline cellulose 0.5 g;
Distilled water q.s. to 1000 ml.
The medium so prepared is autoclaved for 20 min at 121°C.

A single colony of the *Bacillus clausii* ATCC 700160 strain, or 0.15 ml of its glycerol stock at -80°C, is inoculated into 150 ml of the fermentation medium without starch and cellulose. The inoculum is incubated for about 18 hours at 37°C under stirring until the bacterial concentration reaches about 3x10⁸ cfu/ml.

Successively about 50 ml of preinoculum are added to a 2 litres flask containing 1 litre of fermentation medium. The fermentation parameters are:
Stirring speed = 200 ± 10 rpm
Temperature = 37 ± 0.5°C

The fermentation is prolonged for 36 - 48 hours until the end of logarithmic phase of growth before the beginning of the sporulation, with a bacterial concentration of about 4x10⁹ cfu/ml. pH is adjusted to a value of 9.0 ± 0.05 by a 10 N sodium hydroxide solution, then the separation of the bacterial pellet from the growth broth is carried out by centrifugation at 9000 x g a 4°C for 20 min. The growth broth is submitted to the purification process.

The extraction and the purification comprise ultrafiltration by ion exchange chromatography.

In ultrafiltration, 2 kDa Sartocon slice polyethersulfone cartridges or equivalent, having a surface of 0.1 m², are used.

The centrifuged growth broth, containing the preparation, from the preceding step of fermentation, is submitted to tangential filtration using two cartridges having a molecular cut-off limit of 2 kDa with a total surface of about 0.2 m². A Watson-Marlow pump mod. 520U is employed at a flow of about 3.5 l/min, using a suitable support, adjusting the pressure to about 2-2,5 x 10⁵ Pa. The filtration is prolonged until a recovery of 90 vol. % of the initial volume in the permeate fraction.

Used in the purification by ion exchange chromatography are a 50 mM Tris-HCl pH 9.0 ± 0.05 conditioning/collection buffer, a Q-Sepharose™ fast-flow resin, column diameter of 5 cm, column height of 7 cm, linear speed of 210 cm/h, detection wavelength of 280 nm.

The solution from the step of ultrafiltration is directly loaded onto the column in the selected pH conditions to permit the bond of the present proteins to the chromatographic resin, allowing the proteins to be separated from the interesting fraction that is collected upon an increase of the U.V. signal. The preparation is contained in the non retained fraction.

Then, the Q-Sepharose™ column is washed with the conditioning/collection buffer until the absorbance baseline becomes constant during the product collection.

The solution, containing the preparation so purified, is sterilised by filtration on 0.22 µm pore diameter filters and stored at +4°C. The product is stable when stored for 12 months at room temperature as well as at +4°C.

The described process of production and purification of the preparation was carried out using a single colony of the *Bacillus clausii* ATCC 700160 strain. Results obtained are in the Table 2 below.

**Table 2 (Bacillus clausii ATCC 700160)**

| Sample | Volume ml | Proteins mg/ml | Total mg | Total units | Yield % |
|---|---|---|---|---|---|
| | | | | | |
| Fermented | 1000 | 0.99 | 990 | 1485 | |
| Permeated | 900 | 0.47 | 519 | 1100 | |
| Bulk | 1100 | 0.27 | 297 | 891 | 60 |

### EXAMPLE 2

The same procedure described above for production and purification of the preparation was carried out using a single colony of the *Bacillus cereus* ATCC 10702 strain. Results obtained are in the Table 3 below.

**Table 3 (Bacillus cereus ATCC 10702)**

| Sample | Volume ml | Proteins mg/ml | Total mg | Total units | Yield % |
|---|---|---|---|---|---|
| | | | | | |
| Fermented | 1000 | 1.12 | 1120 | 1564 | |
| Permeated | 900 | 0.56 | 504 | 1068 | |
| Bulk | 1100 | 0.31 | 341 | 991 | 63 |

### EXAMPLE 3

The same procedure described above for production and purification of the preparation was carried out using a single colony of the *Bacillus subtilis* ATCC 10783 strain. Results obtained are in the Table 4 below.

**Table 4 (Bacillus subtilis ATCC 10783)**

| Sample | Volume ml | Proteins mg/ml | Total mg | Total units | Yield % |
|---|---|---|---|---|---|
| | | | | | |
| Fermented | 1000 | 0.87 | 870 | 1214 | |
| Permeated | 900 | 0.44 | 396 | 814 | |
| Bulk | 1100 | 0.26 | 286 | 695 | 57 |

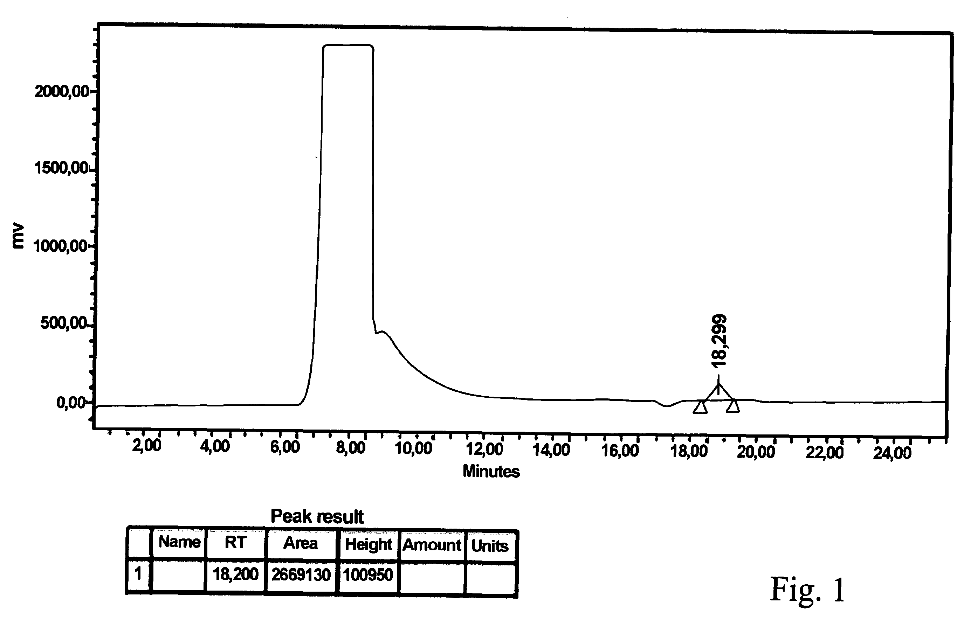

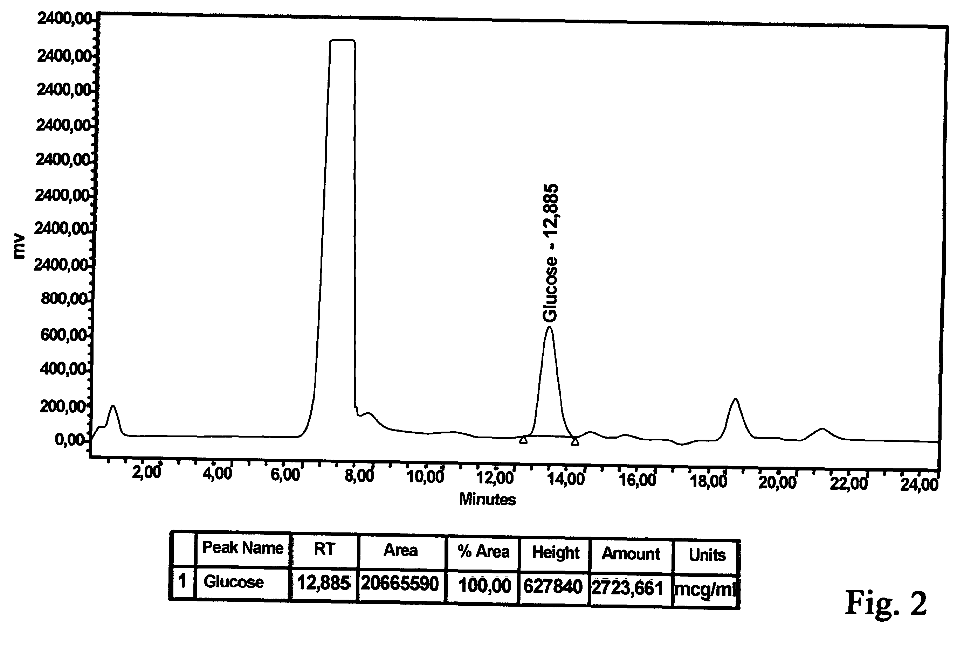

The effect of the different species *Bacillus* is essentially the same and requires both starch and cellulose, as indicated in the following Tables 5 and 6, which show the action of the culture medium on a substrate of *S. mutans* (ATCC 33402 strain), for different time periods of 5 and 180 min, respectively. The results in Table 5 and in Table 6 refer to both the colorimetric test for determining reducing sugars and the checking by HPLC of the same samples for the presence of glucose. By way of example, figure 1 shows the HPLC analysis for the presence of glucose of a sample negative to the colorimetric test, and figure 2 is relevant to a positive sample. The test bacterium relevant to figures 1 and 2 is *Bacillus cereus.*

**Table 5**

| Incubation of *S. mutans* substrate with culture medium for 5 min at 37°C | | | | |
|---|---|---|---|---|
| Bacteria | Fermentation | Fermentation + starch | Fermentation + cellulose | Fermentation + starch & cellulose |
| *B. clausii* | | | | |
| Glucose µg | 0 | 0 | 0 | 2787 |
| Sugars µmoles | 0 | 0 | 0 | 0.3454 |
| *B. subtilis* | | | | |
| Glucose µg | 0 | 0 | 0 | 2277 |
| Sugars µmoles | 0 | 0 | 0 | 0.3111 |
| *B. cereus* | | | | |
| Glucose µg | 0 | 0 | 0 | 2723 |
| Sugars µmoles | 0 | 0 | 0 | 0.3387 |
| *L. reuteri* | | | | |
| Glucose µg | 0 | 0 | 0 | 1930 |
| Sugars µmoles | 0 | 0 | 0 | 0.3015 |

**Table 6**

| Incubation of *S. mutans* substrate with culture medium for 180 min at 37 °C | | | | |
|---|---|---|---|---|
| Bacteria | Fermentation | Fermentation + starch | Fermentation + cellulose | Fermentation + starch & cellulose |
| *B. clausii* | | | | |
| Glucose µg | 0 | 0 | 0 | 2813 |
| Sugars µmoles | 0 | 0 | 0 | 0.3401 |
| *B. subtilis* | | | | |
| Glucose µg | 0 | 0 | 0 | 2705 |
| Sugars µmoles | 0 | 0 | 0 | 0.3289 |
| *B. cereus* | | | | |
| Glucose µg | 0 | 0 | 0 | 2511 |
| Sugars µmoles | 0 | 0 | 0 | 0.3225 |
| *L. reuteri* | | | | |
| Glucose µg | 0 | 0 | 0 | 2110 |
| Sugars µmoles | 0 | 0 | 0 | 0.3131 |

Further the break-down remains unmodified in a pH range between 4.0 and 8.0 (Table 7).

**Table 7**

| Activity on the polysaccharide produced by *S. mutans* of the culture medium with starch and cellulose of different bacteria at various pH (reducing sugars micromoles) | | | | | |
|---|---|---|---|---|---|
| Bacteria | pH 4.0 | pH 5.0 | pH 6.0 | pH 7.0 | pH 8.0 |
| *B. clausii* | 0.3462 | 0.3358 | 0.3769 | 0.3858 | 0.3184 |
| *B. subtilis* | 0.3195 | 0.3036 | 0.3274 | 0.3185 | 0.3214 |
| *B. cereus* | 0.2956 | 0.2866 | 0.3018 | 0.3255 | 0.3203 |
| *Lactobacillus reuteri* | 0.3033 | 0.3125 | 0.3321 | 0.2998 | 0.2881 |

The combinations of various kinds of starch and cellulose maintain the capacity of break-down as shown in Table 8, that indicates the micromoles of reducing sugars released from the *S. mutans* substrate prepared as decribed in literature.

**Table 8**

| | Microcrystalline cellulose | Carboximethyl cellulose | Methyl cellulose | Ethyl cellulose |
|---|---|---|---|---|
| Soluble starch | 0.3955 | 0.3399 | 0.3312 | 0.2856 |
| Rice starch | 0.3211 | 0.2763 | 0.2615 | 0.2565 |
| Potato starch | 0.2987 | 0.3014 | 0.3399 | 0.3101 |
| Corn starch | 0.3114 | 0.2987 | 0.3411 | 0.2935 |

After studies of mixing in different amounts between 0.2 g/l and 2 g/l, the preferred amount of starch and cellulose to be used in the culture medium in the method according to the invention is 0.5 g/l for each of the two components. In fact this concentration allows to obtain a good breaking-down activity towards the polysaccharide produced by the *S. mutans,* and makes much easier the purification process.

Table 9 shows results expressed in micromoles of reducing sugars produced by the fermentation in *Saccharomyces cerevisiae* medium of the *Bacillus clausii* added with various compositions of soluble starch and microcrystalline cellulose. Analogous results are obtained by using other bacterial strains cultivated in different media.

**Table 9**

| Soluble starch g/l | Microcrystalline cellulose (g/l) | | | | | | | |
|---|---|---|---|---|---|---|---|---|
| | **0.2** | **0.3** | **0.5** | **0.8** | **1** | **1.2** | **1.5** | **2** |
| **0.2** | 0.185 | 0.195 | 0.287 | 0.169 | 0.201 | 0.214 | 0.199 | 0.229 |
| **0.3** | 0.178 | 0.270 | 0.335 | 0.228 | 0.199 | 0.222 | 0.214 | 0.208 |
| **0.5** | 0.221 | 0.323 | **0.412** | 0.379 | 0.336 | 0.396 | 0.236 | 0.284 |
| **0.8** | 0.229 | 0.314 | 0.389 | 0.325 | 0.287 | 0.236 | 0.299 | 0.275 |
| **1** | 0.196 | 0.321 | 0.401 | 0.271 | 0.301 | 0.254 | 0.308 | 0.299 |
| **1.2** | 0.214 | 0.289 | 0.412 | 0.269 | 0.299 | 0.245 | 0.333 | 0.304 |
| **1.5** | 0.235 | 0.307 | 0.399 | 0.284 | 0.326 | 0.279 | 0.305 | 0.323 |
| **2** | 0.256 | 0.324 | 0.384 | 0.311 | 0.331 | 0.291 | 0.321 | 0.314 |

Results shown in Table 10 and Table 11 refer to tests on teeth extracted after incubation of 5 and 180 min, and stress how only the broth cultures grown in the presence of starch and cellulose are able to break-down the polysaccharide adhering to teeth. Said break-down does not show remarkable changes between 5 and 180 min of incubation and is confirmed by the presence of glucose, after acid hydrolysis, in the liquid separated from the tooth.

**Table 10**

| Qualitative analysis - Tests on teeth - presence of glucose - 5 min | | | | |
|---|---|---|---|---|
| Bacteria | Standard fermentation | Standard fermentation + starch | Standard fermentation + cellulose | Standard fermentation + starch & cellulose |
| *B. clausii* | negative | negative | negative | positive |
| *B. subtilis* | negative | negative | negative | positive |
| *B. cereus* | negative | negative | negative | positive |
| *Lactobacillus reuteri* | negative | negative | negative | positive |

**Table 11**

| Qualitative analysis - Tests on teeth - presence of glucose - 180 min | | | | |
|---|---|---|---|---|
| Bacteria | Standard fermentation | Standard fermentation + starch | Standard fermentation + cellulose | Standard fermentation + starch & cellulose |
| *B. clausii* | negative | negative | negative | positive |
| *B. subtilis* | negative | negative | negative | positive |
| *B. cereus* | negative | negative | negative | positive |
| *Lactobacillus reuteri* | negative | negative | negative | positive |

Products obtained in examples 1, 2, 3 are submitted to biochemical characterisation by performing tests such as: Sodium Dodecyl Sulphate - Polyacrylamide Gel Electrophoresis (SDS-Page) with Coomassie blue staining, using a Phast System (Amersham), a 8-25% polyacrylamide gel gradient loading 4 microlitre samples (Wyckoff et al. 1987; Jovin, 1973; Wilson 1979; Allen, et al. 1984); HPLC-Rp chromatographic profiling with Source RPC ST 4,6x150 column (Ge-healthcare cod.17-5116-01) with a flow of 1 ml/min using 0.1% aqueous trifluoroacetic acid (TFA) as eluent A and 0.08% TFA in Acetonitrile as eluent B in a 10-35% gradient for 22 min at 40°C, by injecting 100 microlitre samples and detecting results by photodiode array detector at 280 nm; activity test on polysaccharides by determination of reducing sugars (C.R. Harwood and S.M. Cutting "Molecular Biological Methods for Bacillus", John Wiley & Sons 1990, 380-383); bacterial inhibition and bactericidal activity tests; protein assay using BCA method (Smith, P.K. et al. 1985. Anal. Biochem. 150:76-85); determination of the absence of proteases with QuantiCleave Protease Assay Kit Pierce (Bubnis et al., 1992; Habbeb, 1996). Results obtained are summarised in Table 12.

**Table 12**

| **TEST** | *B. clausii* | *B. cereus* | *B. subtilis* |
|---|---|---|---|
| | | | |
| **IDENTIFICATION** | | | |
| Chromatographic profile | According to the standard batch | According to the standard batch | According to the standard batch |
| | | | |
| **Protein concentration** | 0.27 mg/ml | 0.31 mg/ml | 0.26 mg/ml |
| | | | |
| **SDS PAGE** | no protein band in 1 - 98 kDa | no protein band in 1 - 98 kDa | no protein band in 1 - 98 kDa |
| | | | |
| **BACTERIAL INHIBITION** | | | |
| *Escherichia coli* | negative | negative | negative |
| *Streptococcus mutans* | negative | negative | negative |
| *Aspergillus niger* | negative | negative | negative |
| *Pseudomonas* | | | |
| *aeruginosa* | negative | negative | negative |
| *Staphylococcus aureus* | negative | negative | negative |
| | | | |
| **PROTEOLYTIC ACTIVITY** | absent | absent | absent |
| | | | |
| **ACTIVITY ON POLYSACCHARIDES** | | | |
| Dextran from *S*. | | | |
| *mutans* | 3 Units/mg | 2.9 Units/mg | 2.4 Units/mg |
| Pullulan | absent | absent | absent |
| Starch | absent | absent | absent |
| Cellulose | absent | absent | absent |
| Xylan | absent | absent | absent |

### EXAMPLE 4

In this example a process of production of a batch of the preparation in question by employing a 16 litres fermentor and using the *Bacillus clausii* ATCC 700160 strain, for stressing the possibility of industrialisation, is described. The preparation obtained shows all the chemico-physical features already described, with an approximately 30% yield increase. The fermentation medium method is formed of:
*Saccharomyces cerevisiae* (blocks of brewer's yeast) 250 g;
NaCl 50 g ;
Solubile starch 5 g;
Microcrystalline cellulose 5 g;
Distilled water q.s. to 10 l.
10 litres medium are prepared and directly sterilised in the fermentor for 20 min at 121 °C.

A single colony of the *Bacillus clausii* ATCC 700160 strain, or 0.5 ml of its glycerol stock at -80 °C, is inoculated into 500 ml of the fermentation medium without starch and cellulose. The inoculum is incubated for about 18 hours at 37 °C under stirring until the bacterial concentration becomes about 3x10⁸ cfu/ml.

Successively about 500 ml of preinoculum are inoculated under sterile conditions into a 16 litres fermentor containing 10 litres of fermentation medium already sterilised in situ. During the fermentation the parameters listed in Table 13 are monitored.

**Table 13**

| Time | **pH** | **Temperature** | rpm | **Volume** Fermented | Aeration | | **O₂** Dissolved | **O.D.** Optical Density 600 | **cfu** | **%** |
|---|---|---|---|---|---|---|---|---|---|---|
| | | | | | | | | | | |
| H | | °C | | | l/m | v/v/m | % | nm | Ml | Spores |
| **12** | **8.74** | **37** | **600** | **10** | **2** | **20** | **98** | **0.8** | **2.2 x10⁷** | **0** |
| **24** | **8.5** | **37** | **600** | **10** | **2** | **20** | **88** | **2.2** | **2 x10⁸** | **0** |
| **36** | **7.5** | **37** | **600** | **10** | **2** | **20** | **92** | **4** | **8 x10⁸** | **0** |
| **48** | **6.7** | **37** | **600** | **10** | **2** | **20** | **94.5** | **6.3** | **4.6 x10⁹** | **10** |

The fermentation is prolonged for 36 - 48 hours until the end of the logarithmic phase of growth before the beginning of sporulation, with a bacterial concentration of about 4x10⁹ cfu/ml. pH is adjusted to a value of 9.0 ± 0.05 by 10 N sodium hydroxide solution, then the separation of the bacterial pellet from the growth broth is carried out by centrifugation at 9000 x g at 4°C for 20 min. The growth broth is submitted to the purification process.

The extraction and the purification comprise ultrafiltration by ion exchange chromatography.

In ultrafiltration, 2 kDa Sartocon slice polyethersulfone cartridges or equivalent, having a surface of 0.7 m² are used.

The centrifuged growth broth, containing the preparation, from the preceding step of fermentation, is submitted to tangential filtration using two cartridges having a cut-off of 2 kDa with a total surface of about 1.4 m² and a Sartocon Support 2. A Watson-Marlow pump mod. 520U/REM is employed at a flow of about 600 ml/min, adjusting the pressure to about 2-2.5 x 10⁵ Pa. The filtration is prolonged until a recovery of 90 vol. % of the initial volume in the permeate fraction. The filtration time is about 6 hour.

Used in the purification by ion exchange chromatography are a 50 mM Tris-HCl pH 9.0 ± 0.05 conditioning/collection buffer, a Q-Sepharose™ fast-flow resin, column diameter of 5 cm, column height of 15 cm, linear speed of 450 cm/h, detection wavelength of 280 nm, flow of 150 ml/min The purification time is about 2 hours.

The remaining part of the process is the same to that in example 1. Results obtained are contained in the next Table 14.

**Table 14 (Bacillus clausii ATCC 700160 with fermentor)**

| Sample | Volume ml | Proteins mg/ml | Total mg | Total units | Yield % |
|---|---|---|---|---|---|
| | | | | | |
| Fermented | 10000 | 0.93 | 9300 | 18113 | |
| Permeated | 9000 | 0.53 | 4830 | 11040 | |
| Bulk | 11000 | 0.39 | 4299 | 13775 | 76 |

The preparation obtained has been characterised as described for the preceding examples since it does not depart from the parameters. Further, the reproducibility of the process has been evaluated, by repeating it three times. Results obtained, as indicated in Table 15, show a clear reproducibility and possibility of industrial use of the process.

**Table 15 Reproducibility**

| Fermentation | Volume ml | Total mg | Total units | Yield % |
|---|---|---|---|---|
| | | | | |
| 1 | 11000 | 4299 | 13775 | 76 |
| 2 | 10500 | 4170 | 12151 | 68 |
| 3 | 11000 | 4418 | 13014 | 70 |

According to the method of the invention a preparation of biological origin is obtained able to remove the polysaccharide adhering to the tooth surface and consequently the bacterial biofilm, in humans and animals. The preparation, probably a polypeptide mixture, has been applied suitably directly to the tooth surfaces. The preparation has shown to be especially important for individuals affected by diseases associated with bacterial infection of oral cavity. A further use of the preparation relates to the field of the dental implantology as a co-adjuvant and/or medicament. It is also possible to administer the preparation associated with other active principles, for example antibacterial drugs like Chlorhexidine, anti-inflammatory or others for oral hygiene, in the form of suitable preferably topical composition. The preparation can also be used in a composition containing excipients and/or adjuvant and/or vehicles and/or hydrating agents and/or preservatives.

Some preferred examples of excipients and hydrating agents are isotonic agents, sugars, polyalcohols, buffers, chelating agents, antioxydants and antibacterials.

Preferably, the preparation is designed for topical application, in emulsion, gel, cream, mouthwash, toothpaste, chewing gum and tablets also in retard form, with sustained release, or any other formulation considered suitable for the application, and that may contain optionally flavours and/or colours.

In case an emulsion or suspension is prepared, the composition may also contain surfactants commonly used in the preparation of medicaments. Preferably water/oil emulsions are used as basis.

In case a gel is prepared, the composition may contain gelling agents like starch, glycerol, polyethylene, polypropylene glycol, poly(meth)acrilate, isopropyl alcohol and hydroxistearate, polyvinylpyrrolidone, carboxymethylcellulose, etc. The composition may also be prepared in the form of a spray. The main advantage of a spray formulation is to maximise the surface contact of the composition with the site requiring the therapeutic activity. The suitable dosage depends on the formulation employed. For topical preparations, the amount used as active principle is preferably between 0.01 and 10 mg per gram or millilitre of composition used. It is preferable to apply said topical composition 1 - 4 times per day. The use of the preparation according to the invention is analogous to that of any other co-adjuvant product for oral hygiene and is designed to be daily.

The preparation may also be used adhering or immobilised in a plaster and/or strips, and/or dental bite. The preparation is designed to remain on plaster, strips, or bite and exerts its activity when released.

In particular, experimentally the following topical composition has been prepared.

An aliquot of purified preparation (as described in examples 1, 2, 3) is diluted to concentrations of 0.05 mg/ml, 0.1 mg/ml, 0.15 mg/ml, and of 0.25 mg/ml in a solution containing the following components: 20 mg/ml propylenglycol + 300 mg/ml sorbitol + 10 mg/ml Carbomer 940 + 0.01M sodium acetate + 0.3 mg/ml methylparaben. pH is adjusted to 6.0 using 10 N sodium hydroxide solution.

The efficacy of the preparation was determined as breaking down activity of the polysaccharide produced by the *Streptococcus mutans* (ATCC 33402 strain) cariogenic bacterium grown in a suitable medium containing sucrose (Kreth J et al. Oral Microbiol. Immunol. 2008; Duarte S. et al. Oral Microbiol. Immunol. 2008). At the end of *Streptococcus mutans* fermentation, the medium is centrifuged for eliminating completely the culture broth, then at least three washings are carried out with water until the complete elimination of the sucrose (checked by HPLC analysis, using a Ca 300x6.5 mm Carbohydrates column, by eluting with water for HPLC and refractive index detector). The presence of the polysaccharide chain in the substrate prepared in this way, is checked with the same methodology by detecting the presence of glucose after acid hydrolysis for 12 hours at 100°C. The substrate produced is used for determining the breaking-down activity relevant to the samples. The compositions analysed and the concentrations relevant to the preparation according to the invention are those referred to in the topical composition, and the control is constituted by the sole vehicle in which the preparation according to the invention has been dissolved (Table 16). The test has been repeated also in the presence of saliva at 37°C (Table 17), by simulating the conditions of the oral cavity, to verify that the activity of the preparation according to the invention remains unchanged.

**Table 16**

| **Substance** | **Concentration** | **Dosage** | **Reducing sugars micromoles** | | | | | |
|---|---|---|---|---|---|---|---|---|
| | | | *B. clausii* | | *B. cereus* | | *B. subtilis* | |
| | | | TIME | | | | | |
| | | | 5 min | 10 min | 5 min | 10 min | 5 min | 10 min |
| | | | | | | | | |
| Control | 0 mg/ml | 1 g | 0.00 | 0.00 | 0.00 | 0.00 | 0.00 | 0.00 |
| | | | | | | | | |
| Sample 1 | 0.05 mg/ml | 1 g | 0.05 | 0.06 | 0.10 | 0.11 | 0.09 | 0.09 |
| | | | | | | | | |
| Sample 2 | 0.10 mg/ml | 1 g | 0.13 | 0.13 | 0.14 | 0.13 | 0.11 | 0.12 |
| | | | | | | | | |
| Sample 3 | 0.15 mg/ml | 1 g | 0.19 | 0.18 | 0.17 | 0.17 | 0.16 | 0.16 |
| | | | | | | | | |
| Sample 4 | 0.25 mg/ml | 1 g | 0.25 | 0.25 | 0.27 | 0.28 | 0.26 | 0.27 |

**Table 17**

| **Substance** | **Concentration** | **Dosage** | **Reducing sugars micromoles** | | | | | |
|---|---|---|---|---|---|---|---|---|
| | | | *B. clausii* | | *B. cereus* | | *B. subtilis* | |
| | | | TIME | | | | | |
| | | | 5 min | 10 min | 5 min | 10 min | 5 min | 10 min |
| | | | | | | | | |
| Control | 0 mg/ml | 1 g | 0.00 | 0.00 | 0.00 | 0.00 | 0.00 | 0.00 |
| | | | | | | | | |
| Sample 1 | 0.05 mg/ml | 1 g | 0.07 | 0.08 | 0.10 | 0.10 | 0.08 | 0.09 |
| | | | | | | | | |
| Sample 2 | 0.10 mg/ml | 1 g | 0.12 | 0.13 | 0.12 | 0.13 | 0.11 | 0.11 |
| | | | | | | | | |
| Sample 3 | 0.15 mg/ml | 1 g | 0.18 | 0.18 | 0.16 | 0.17 | 0.15 | 0.17 |
| | | | | | | | | |
| Sample 4 | 0.25 mg/ml | 1 g | 0.23 | 0.24 | 0.25 | 0.24 | 0.22 | 0.22 |

The HPLC analysis of the samples positive to the reducing-sugar test has shown the presence of glucose confirming that the break-down of the polysaccharide took place.

The test directly carried out on the polysaccharide produced by the cariogenic bacterium (isolated from the caries) present in the oral cavity, also in presence of saliva, showed that break-down activity on the polysaccharide chain was produced by the *Streptococcus mutans,* at all the concentrations examined as compared with the control. In particular, also at the lowest concentration tested an activity is obtained in very short times, being compatible with the types of use as indicated. As shown in Table 17 the activity remains unchanged also in presence of saliva.

The activity test of the preparation in question has been carried out also on extracted teeth, after cleaning and sterilisation. The teeth have been removed in a sterile hood and, under sterile conditions, incubated in a *Streptococcus mutans* culture in conditions favourable to the formation of the bacterial biofilm for about 48 hours.

At the end of the incubation the teeth have been taken and dried at 35°C for 30 min and then individually stained using Red- Cote GUM- Sunstar Americas staining tablets for bacterial plaque dissolved in water (1 tablet in 10 ml).

An aliquot of purified preparation according to the invention (as described in examples 1, 2, 3) is diluted to concentrations of 0.15 mg/ml, and of 0.25 mg/ml in a solution containing the following components: 20 mg/ml propylenglycol + 300 mg/ml sorbitol + 10 mg/ml Carbomer 940 + 0.01M sodium acetate + 0.3 mg/ml methylparaben. pH is adjusted to 6.0 using 10 N sodium hydroxide solution.

Successively, the gel produced has been transferred in 20 g tubes; by using the same procedure some control tubes have been prepared to be used in the experiment.

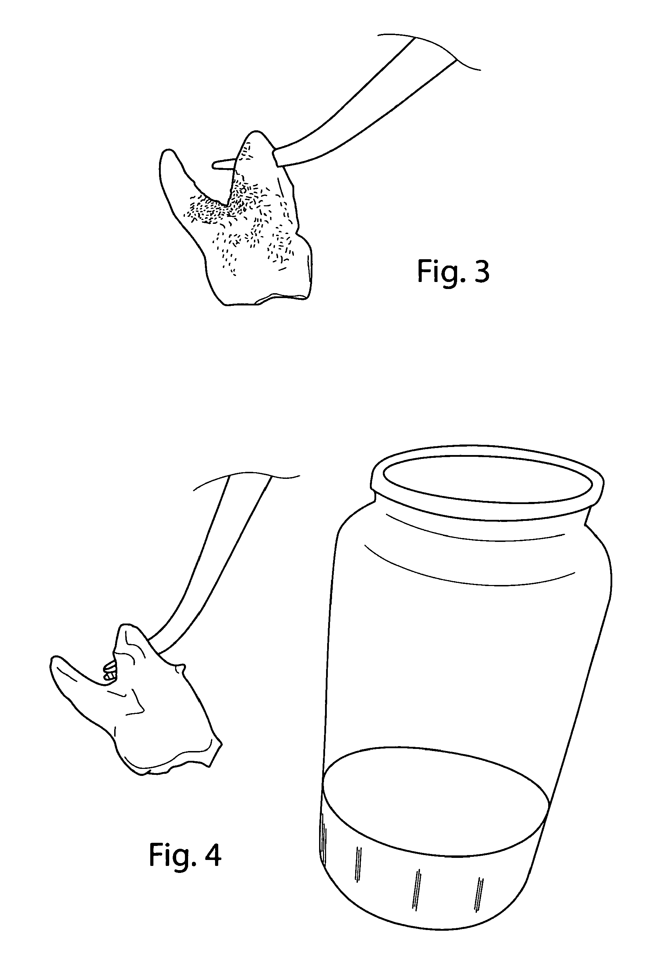

The tooth prepared and stained as before described has been washed by immersion in water for removing the stain in excess. Before testing the tooth by the preparation, the tooth has been immersed in control for 10 min at 37°C in order to check for stain loss, if any, due to the components of the gel. Next, after removing the control with care, the tooth has been immersed in the preparation to be tested for 5 and 10 min at 37 °C, taking pictures of the results obtained at different times. The results, that are shown in figures 3 and 4 and represent the status of the tooth before treatment and after a 5 min treatment, have been analysed visually for attributing a numerical value of plaque removal. Arbitrarily, a value 100 was attributed to the tooth after staining and a percent of removal has been given in relation to decrease in stain intensity. The compound released form the tooth surface has been submitted to acid hydrolysis and analysed in HPLC for confirming the presence of glucose. For shortness sake, relevant chromatograms are not reported. As evident from Table 18, the control does not exhibit any removal activity, whereas the two different concentrations tested exhibit a high removal activity already after 5 min.

**Table 18**

| **Substance** | **Concentration** | **Dosage** | **Percent of plaque removal** | | | | | |
|---|---|---|---|---|---|---|---|---|
| | | | *B. clausii* | | *B. cereus* | | *B. subtilis* | |
| | | | TIME | | | | | |
| | | | 5 min | 10 min | 5 min | 10 min | 5 min | 10 min |
| | | | | | | | | |
| Control | 0 mg/ml | 2 g | 0.00 | 0.00 | 0.00 | 0.00 | 0.00 | 0.00 |
| | | | | | | | | |
| Sample 1 | 0.15 mg/ml | 2 g | 65 | 70 | 55 | 70 | 55 | 70 |
| | | | | | | | | |
| Sample 2 | 0.25 mg/ml | 2 g | 85 | 90 | 85 | 90 | 80 | 85 |

In conclusion, although in state of the art it is known that the addition of one or more components during the fermentation step of bacteria of genus *Bacillus,* is able to induce the production of substances selectively acting on desired substrates, it is evident that only the combined addition of both starch and cellulose is able to act on the metabolism of the same bacteria inducing them to produce a preparation able to break down the polysaccharide produced by *S. mutans.* In fact, the preparation obtained by the method according to the invention does not exhibit any inhibition activity in the growth of *S. mutans* and is not able to inhibit the synthesis of the polysaccharide produced by the bacterium. Said preparation is not constituted of proteins having a molecular weight higher than 2 kDa and does not exhibit a typical enzymatic activity, does not exhibit activity changes in a range of pH between 4.0 and 8.0, has neither bactericidal activity or activity inhibiting other bacterial species. The breaking down capacity of the polysaccharide produced by the *S. mutans* cariogenic bacterium does not depend on the type of the *Bacillus* strain used or on the culture medium in which it is fermented and thus the breaking down capacity cannot be ascribed to substances already known in the art, as said activity is absent without adding together starch and cellulose, probably because the production of a compound is induced that otherwise would not be produced.

## Claims

1. A method of obtaining a preparation of biological origin for oral hygiene to prevent the onset of tooth decay, comprising steps of fermentation of bacteria in a culture medium, separation of the same and purification of the culture medium in which the bacteria have been cultivated, **characterised in that** the step of fermentation of bacteria includes a combined addition of both starch and cellulose to the culture medium to the final concentration in the culture medium between 0.2 g/l and 2 g/l.

2. The method according to claim 1, in which the amount of starch and the amount of cellulose expressed in weight with respect to the final volume of the culture medium are preferably 0.5 g/l each.

3. The method according to claim 1, in which the culture medium is constituted of *Saccharomyces cerevisiae* (brewer's yeast), starch and cellulose.

4. The method according to claim 1, in which the starch is chosen from the group comprising rice starch, corn starch, and potato starch.

5. The method according to claim 1, in which the cellulose is chosen from the group comprising microcrystalline cellulose, methyl cellulose, carboxymethyl cellulose, and ethyl cellulose.

6. The method according to claim 1, in which the cultivated bacteria are of genus *Bacillus.*

7. The method according to claim 6, in which the bacteria of genus *Bacillus* are chosen among those of the species *clausii, cereus,* and *subtilis.*
